# EUROPEAN PATENT APPLICATION

(11) **EP 0 835 649 A1**
(43) Date of publication of application: **15.04.1998**
(21) Application number: 97305845.6
(22) Date of filing: 01.08.1997
(51) Int. Cl.: A61K 7/15, A61K 7/48

(54) **Shaving aid with complexing agent**

(30) Priority: 04.09.1996 US 707437
(71) Applicant: WARNER-LAMBERT COMPANY, Morris Plains New Jersey 07950 (US)
(72) Inventor: Vreeland, William Elbert, Shelton, Connecticut 06484 (US)
(74) Representative: Powell, Timothy John

(57) **Abstract**

A shaving aid for use with a wet razor comprising a water sensitive complexing agent, e.g., a cyclodextrin, and at least one active ingredient. According to this embodiment of the present invention, the active ingredient may be selected from a wide variety of active ingredients.

## Description

The present invention is directed to shaving aids and, more particularly, to shaving aids comprising a novel delivery system comprising at least one water-sensitive complexing agent.

Various types of shaving aids are known in the razor industry. One popular type of shaving aid contains at least one water insoluble polymeric matrix and at least one water soluble material which exudes out of the insoluble matrix upon contact with water during shaving. Such shaving aids can be molded or extruded and then attached to razor heads mechanically, for example, with snap-in fittings or by molding the shaving aid into recesses containing anchors in a razor head. Such shaving aids are designed to exude the water soluble materials from the insoluble porous micro-structure when the shaving aid becomes wet during shaving.

A number of different materials have been suggested for use as shaving aids. Previously suggested active ingredients include those disclosed in U.S. Patent No. 4,170,821 to Booth, which is hereby incorporated by reference, and which can be used alone or in various combinations, such as:
A. A lubricating agent for reducing the frictional forces between a razor and the skin, e.g., a micro-encapsulated silicone oil.
B. An agent which reduces the drag between the razor parts and the shaver's face, e.g., a polyethylene oxide in the range of molecular weights between 100,000 and 6,000,000; a non-ionic polyacrylamide; and/or a natural polysaccharide derived from plant materials such as "guar gum".
C. An agent which modifies the chemical structure of the hair to allow the razor blade to pass through the whiskers very easily, e.g., a depilatory agent is one example.
D. A cleaning agent which allows the whisker and skin debris to be washed more easily from the razor parts during shaving, e.g., a silicon polyethylene oxide block copolymer and detergent such as sodium lauryl sulphate.
E. A medicinal agent for killing bacteria, or repairing skin damage and abrasions.
F. A cosmetic agent for softening, smoothing, conditioning or improving the skin.
G. A blood coagulant for the suppression of bleeding that occurs from nicks and cuts.
H. An astringent for constricting blood vessels thereby stemming the flow of bodily fluids such as lymph which may exude from skin which has been irritated during shaving.
Alternatively, the shaving aid may comprise one or more of the shaving aids disclosed in U.S. Patent No. 5,056,221 to Thoene, U.S. Patent No. 4,044,120 to Rowsell et al., U.S. Patent No. 5,095,619 to Davis el al., or Japanese Patent Application No. Hei 7 [1995] - 24156 to Miyazaki, et al. which are also hereby incorporated by reference.

During manufacturing, particularly during molding or extrusion of a shaving aid, certain active ingredients, particularly oil soluble ingredients such as Vitamin E, essential oils, fragrances, etc., can become trapped within the water-insoluble phase of the shaving aid. The active ingredient will then be "tied-up" in the water-insoluble phase and will not be released during shaving.

While it is desirable in certain instances to provide shaving aids with active ingredients including oil soluble additives such as vitamin E, essential oils and fragrances, as well as other such additives, it has been found that during processing, certain additives can become trapped within a water insoluble phase of the shaving aid. If these additives are trapped, they will not be released during shaving and their intended benefits will not be realized.

According to the invention, there is provided a shaving aid as defined in Claim 1 or Claim 12; and a method of forming a shaving aid as defined as defined in Claim 13. Further, advantageous features of the invention are defined in the dependent claims.

Thus there is advantageously provided a new delivery system for active ingredients which protects the active ingredients during processing so that they are not inextricably bound to the shaving system.

There is also advantageously provided a delivery system which protects the active ingredients during processing in order to minimize the risks of degradation of further reduction in efficacy due to processing.

One preferred embodiment of the present invention comprises a shaving aid for use with a wet razor comprising a water sensitive complexing agent, e.g., a cyclodextrin, and at least one active ingredient. According to this embodiment of the present invention, the active ingredient may be selected from a wide variety of active ingredients.

It has now been found that the potential for an active ingredient to become trapped by the water insoluble phase of a shaving aid or to otherwise have its efficacy reduced can be avoided by first protecting the active ingredient with a complexing agent which will protect the active ingredient upon contact with water during shaving.

There now follows a description of preferred embodiments of the invention, by way of example.

While shaving aids are most commonly attached directly to a razor head, it is also possible to utilize a shaving aid which is separate from a razor head. As used herein, the term "razor head" is meant to include both the operative section of disposable razors as well as disposable cartridges design for attachment to a separate razor.

The term "shaving aid", as used herein, refers equally to either the active ingredient alone or to the active ingredient combined with one or more other materials, such as a complexing agent or a water-insoluble micro-porous matrix structure.

Cyclodextrins are a class of cavity-containing cyclic compounds possessing the property of forming a type of complex known as molecular inclusion complex. Such complexes provide a method of anchoring or entrapping another chemical compound known as the "guest" compound, within the complexing agent which is known as the "host", without the formation of covalent bonds. Cyclodextrins are a group of cyclic, non-reducing oligosaccharides comprising six, seven or eight glucopyranose rings, respectively known as alpha, beta, and gamma cyclodextrins. Cyclodextrins exhibit high thermal stability, with a decomposition temperature approaching 3000C. Cyclodextrins are able to molecularly encapsulate and thereby modify the apparent physical and chemical properties of guest molecules. It has been found that cyclodextrins will form inclusion complexes which prevent subject active ingredients from becoming trapped by an insoluble porous micro-structure of a shaving aid. Furthermore, when the resulting shaving aids become wet during shaving, typically with water having a temperature of about 700 - 1200F, the cyclodextrin will release the guest molecule render the active ingredient available to provide its intended effect.

Cyclodextrin inclusion is a molecular phenomenon in which usually only one guest molecule interacts with the cavity of a cyclodextrin molecule to become entrapped, unlike other previously described micro-encapsulation in which more than one guest molecule is physically entrapped in an encapsulation matrix. In order to form a cyclodextrin complex, one guest molecule must come into contact with the cyclodextrin cavity to form a stable association. A variety of non-covalent forces, such as van der Waal forces, hydrophobic interactions and other forces, are responsible for the formation ofa stable complex.

In the case of some low molecular weight molecules, more than one guest molecule may fit into the cavity. In the case of some high molecular weight molecules, more than one cyclodextrin molecule may bind to the guest. Only a portion ofthe molecule must fit into the cavity to form a complex, which is the case with many high molecular weight molecules. As a result, a one to one molar ratio is not always achieved, especially with high or low molecular weight guests and some preliminary complex formation and analysis may be needed to determine relative amounts of cyclodextrin and guest to be added for complexation.

In the crystalline form, only the surface molecules of the cyclodextrin crystal are available for complexation. In solution, more cyclodextrin molecules become available for complexation.

The guest molecule has an influence on the solubility of the complex being made. Guests having a charged group which will extend out of the cavity after complexation may form soluble complexes. The charged group is easily solvated by water and the portion complexed in the cyclodextrin is generally apolar and not very soluble in water. Complexed in the cyclodextrin, this portion has the advantage of water solvation because of the hydroxyl groups on the outside of the cyclodextrin molecule.

Heating is commonly used to form complexes. Heat can be used to increase the solubility of the cyclodextrin and the guest to put individual molecules into solution so that complexes can be formed. This increases the probability of a guest molecule and a cyclodextrin molecule being able to come together to form a complex. Cooling of the solution may be necessary to crystallize or precipitate the complexes in order to allow formation of a stabile complex or to decrease the solubility ofa soluble complex.

Water is the most commonly used solvent in which complexation reactions are performed. The more soluble the cyclodextrin is in the solvent, the more molecules that are available for complexation. The guest must be able to displace the solvent from the cavity if the solvent complexes with the cyclodextrin. In the case of water; water is very easily displaced. The solvent must be easily removed if it is desirable to obtain complexes free of solvent. In some cases the guest to be complexed is a solvent or in the case of multicomponent guests, one of the components may act as a solvent and be included as a guest.

Not all guests are readily solubilized in water making complexation either very slow or impractical. In such cases, use of an organic solvent to dissolve the guest can be desirable. The solvent of choice should be a solvent which does not complex well with cyclodextrin, which is easily removed for example by evaporation and in which the guest is highly soluble so that only a small amount of solvent is used. Ethanol and dimethyl ether are examples of such solvents. Upon addition ofthe dissolved guest to the cyclodextrin solution, the guest may be solubilized by addition ofthis small amount of solvent allowing complexation to occur easily, or the guest may be dispersed as a fine precipitate. In the latter case, a long stirring or complexation time may be needed, but complexation occurs more rapidly than if the guest were still in the form of large crystals. It is possible to add sufficient solvent, such as ethanol, to water to allow solubility of both the cyclodextrin and guest. Cyclodextrins reach maximum solubility in approximately 30% ethanol. Using a system such as this, some guests are complexed very well since they react strongly enough to displace the ethanol from the cavity while some guests will partition between the cavity and solution phases so that complexation is not complete.

As the amount of water is increased, the amount of cyclodextrin and the amount of guest that can be solubilized increases so that more of these molecules exist in a molecular form so that they can complex more readily. As the amount of water is increased further, the cyclodextrin and guest may become sufficiently dilute so that they do not come into contact with each other as frequently as in a more concentrated solution. Water molecules will have a greater probability of occupying the cavity of the cyclodextrin than the guest. With most guests, this effect is minimal but can have a great effect on more soluble guest. With these guests, use of less water is beneficial to increase the amount of guest in the cavity during complexation. It is also desirable to keep the amount of water sufficiently low to exceed the solubility of the complex. More guest is released more readily from the complex in the soluble state than in the solid or precipitated state.

Some high molecular weight compounds such as some oils have a tendency to associate with themselves rather than the cyclodextrin. Often in such cases, more water with good mixing will allow better dispersion and separation of the oil molecules or isolation of the oil molecules from each other. When the oil molecules come into contact with the cyclodextrin, they will form a more stable complex than they would if less water was present.

Volatile guests can be lost during complexation, especially if heat is used. With highly volatile guests, loss can be minimized by using a sealed container. The guest in the aqueous phase will complex and as it complexes guest in the gas phase will dissolve in the aqueous phase. With less volatile guests refluxing can also be used to prevent loss of the guest. This can be done with a reflux column or on a small scale using an Erlenmeyer flask and allowing the guest to condense on the sides and cover ofthe flask.

### Complexation Techniques

There are several techniques and variation of these techniques used to form cyclodextrin complexes. The utility of a technique will be determined by such factors as the amount of complex to be formed, limitations that may be imposed because of stability factors of the guest, and ease of recovery ofthe complex.

### Coprecipitation

Cyclodextrin is dissolved in water and the guest is added while stirring the cyclodextrin solution. The concentration of the host, e.g., beta cyclodextrin, is dissolved in water and the guest is added while stirring the cyclodextrin solution. The concentration of beta cyclodextrin can be as high as approximately 20% if the guest can tolerate higher temperatures. Higher concentration can be achieved with the more soluble cyclodextrin. The concentration is chosen to be sufficiently high so that the solubility of cyclodextrin guest complex will be exceeded as the complexation reaction proceeds or as the reaction cools. In many cases, the solution of cyclodextrin and guest must be cooled while stirring before a precipitate forms.

The precipitate formed can be collected by decantation, centrifugation or filtration. The precipitate may be washed with a small amount of water or other water miscible solvent such as ethyl alcohol, methanol or acetone. This may be detrimental with some complexes so that testing should be done before scaling up too far.

This method has the advantage that it can be easily done in the laboratory and one can generally obtain assurance of complex formation by the formation of precipitate. The precipate forms at a temperature higher than that temperature where crystallization of uncomplexed cyclodextrin takes place and has an appearance different from beta cyclodextrin crystal. Generally crystals or the precipitate of the complex are smaller than the cyclodextrin crystal. In order to minimize tank capacity, water usage, time and energy for heating and cooling, the mother liquor may be used for more than one batch of complex.

### Slurry Complexation

It is not necessary to dissolve the cyclodextrin completely to form a complex. Cyclodextrin can be added to water as high as 50 to 60% solids and stirred. The aqueous phase will be saturated with cyclodextrin in solution. Guest molecules will complex with the cyclodextrin in solution and as the cyclodextrin complex saturates the water phase, the complex will crystallize or precipitate out of the aqueous phase. The cyclodextrin crystals will dissolve and continue to saturate the aqueous phase to form the complex and precipitate or crystallize out of the aqueous phase. The complex can be collected in the same manner as with the coprecipitation method.

The amount of time required to complete the complexation is variable and depends upon the guest. Assays must be done to determine the amount of time required. Generally slurry complexation is performed at ambient temperature. With many guests, some heat may be applied to increase the rate of complexation, but care must be applied since too much heat can destabilize the complex and the complexation reaction may not be able to take place completely. This must be evaluated for each guest.

The main advantage of this method is the reduction of the amount of water used and size of the reactor. The amount of heat energy used may be reduced but this and reactor time are dependent upon guest and its rate of complex formation.

### Paste complexation

This is a variation of the slurry method. Only a small amount of water is added to form a paste which is mixed with the cyclodextrin using a mortar and pestle or on a large scale using a device such as a ball mill or other mixer designed for handling thick pastes. The amount of time required is dependent upon the guest.

Pastes will sometimes dry forming a hard mass instead of a fine powder. This is dependent upon the guest and the amount of water used in the paste. Generally the hard mass can be dried thoroughly and milled to obtain a powdered form ofthe complex.

### Damp Mixing and Heating

This method uses little or no added water. Variations in time and temperature may be desired, depending upon the guest.

### Extrusion

Extrusion is a variation of the heating and mixing method and is a continuous system. Cyclodextrin, guest and water can be premixed or mixed as added to the extruder. The degree of mixing, amount of heating and time can be controlled in the barrel of the extruder. Depending upon the amount of water, the extruded complex may dry as it cools or the complex may be placed in an oven to dry.

Extrusion has the advantages of being a continuous process and uses very little water. Due to the heat generated, some hot labile guests may be decomposed using this method.

### Dry Mixing

Some guests can be complexed by simply adding guest to the cyclodextrin and mixing them together. This works best with oils or liquid guests. The amount of mixing time required is variable and depends upon the guest.

Generally this method is performed at ambient temperature. This method is a variation of the paste method.

### Drying of Complexes

Drying can be a critical stage especially with volatile guests since guest can be lost. Drying can be done in an oven, fluid bed dryer or other dryer. Enough water may be present in the filter cake obtained to destabilize the complex as it is heated. Since water evaporates at 1000C, this is the preferred temperature for drying the complex. Generally this temperature can be used for complexes where the boiling temperature of the guest is above 1000C with little or no loss of the complexed guest. Excess or uncomplexed volatile guest will be evaporated, but not the complexed guest. With more guests, there is no further loss of guest once the water has evaporated even if the complex remains in the oven.

### Highly Volatile Guests

For guests with boiling temperatures below 1000C, a lower temperature must be used. There is loss of guest during drying but this loss can be minimized by drying at a temperature at or a few degrees below the boiling point, the amount of loss increases and increases as the number of degrees greater or less than the boiling point increases.

### Low Temperature Drying

A desiccator or freeze dryer may be used to dry complexes. The temperature can be kept low and by saturation of the atmosphere with volatile guest, loss of extremely volatile guest can be minimized. With phosphorous pentoxide as the desiccant, drying time is shorter than with calcium chloride and there is generally less loss of guest. Phosphorous pentoxide will react with a few guest molecules and can result in loss of guest during drying. Freeze drying is especially useful for heat labile guests. Some volatile guests will be lost during freeze drying due to lowering of the boiling point caused by the vacuum.

### Release

Once a complex has been formed and dried, it is very stable and will tend to remain stable indefinitely at ambient temperatures under dry conditions. Displacement of the complexed guest by another guest or heating is required to release the complexed guest. During shaving, water can serve as the guest to replace a complexed guest.

When a complex is placed in water, two steps are involved in the release of the complexed guest. First, the complex must be dissolved and the rate of dissolution is different for each guest complex The second step is the release of the complexed guest as water molecules displace the complexed guest. This rate will also vary with each different guest molecule.

The amount and rate of guest released can be increased by adding another potential guest to the water containing the complex. As the amount of competing guest is added, the amount of guest released increases. Heating will also increase the amount and rate of release.

In the case of complexes containing multiple guest components or cyclodextrin types, guest molecules are not necessarily released in the same proportion as in the original guest mixture. Each guest complex may have different solubilities and rate of release from the complex. If release rates are different for each component, one may be able to compensate by alteration of the guest formation to obtain release in the proper proportion. Addition of a competing guest will also result in release ofthe intended guests.

While the active ingredient/cyclodextrin complexes of the present invention may be formed utilizing different processes, the following examples are provided for purposes of illustration.

### Preparative Method for Cyclodextrin Complexes using Beta-Cyclodextrin

1. Prepare an aqueous suspension of Beta Cyclodextrin (mol. wt., 1135 g/mol) at 10 to 15% concentration (wt/v).
2. Warm the mixture to 650C or until a clear solution is attained.
3. Add a 1:1 molar ratio ofthe guest (based upon the moles ofBeta CD) as a neat liquid or as a suspended material. A precipitate is usually observed at this point. If the liquid is of high viscosity, or is a solid material, it may be desirable to slurry the guest in a minimum amount of acetone or ethanol. This aids in the dissolution of the guest and increases the efficiency of the complexation reaction. The solvent is usually not a concern because it is either in the aqueous phase or is removed during the drying process.
4. Remove the mixture from the heat source and continue stirring until it cools to ambient temperature. The formation of a precipitate is an indication that a complex has formed.
5. Harvest the complex *via* vacuum filtration. One may wash the filter cake with the mother liquor if desired. Washing is sometime done in an attempt to remove any "uncomplexed" materials.
6. Dry the filter cake in a convection oven. 100 - 1050C is usually a suitable temperature.
7. Assay the complex by one ofa number of suitable methods:
   Gas Chromatography - good for a solvent extractable guest.
   HPLC - good for a solvent extractable guest or a soluble complex.
   UV-Vis - good for a complex soluble in DMF or other suitable solvent.

### Complexes Prepared with Hydroxypropyl Beta Cyclodextrin

1. Prepare an aqueous solution of Hydroxypropyl Beta-Cyclodextrin.
   CAVITRONÔ HPBCD, available from American Maize-Products Company of Hammond, Indiana, has a molecular weight of approximately 1540; CAVITRONÔ HPBCD Technical grade has a mol. wt. of approximately 1360; other brands will vary in molecular weight.
2. Add a 1:1 molar ratio ofyour guest to HPBCD.
3. Heat may assist in enhancing the rate of solubilization and complexation.

In general, complexation occurs in a one to one fashion (one mole of guest for each mole of cyclodextrin), however, there are exceptions. Also, with HPBCD, the amount of material which can be solubilized may not be linear with the concentration of cyclodextrin. However, the 1:1 correlation is usually followed, especially at lower concentrations and for guest molecules with a molecular weight below 300 amu's.

### Complexation of Vitamin E

A paste of beta-cyclodextrin (BCD) was made at room temperature. Vitamin E (di-alpha Tocopheryl Acetate) was added dropwise to the BCD paste during 40 minutes with forceful kneading. The mixture was then dried under vacuum over phosphorous pentoxide.

A sample ofthe complex was dissolved in 50% aqueous ethanol and further diluted with ethanol. The amount of Vitamin E was determined by measuring U.V. absorbance at 282 nm against a standard Vitamin E sample.

The complex contained 13.2 grams of Vitamin E per 100 grams. Its spectrum showed no sign of decomposition.

In addition to one or more active ingredients and a complexing agent, the shaving aids of the present invention may also comprise other compounds or blends of compounds such as water-insoluble polymers such as polystyrene and polypropylene. In order to obtain the benefits of the various embodiments ofthe present invention, it is not necessary that all active ingredients or all portions ofthe active ingredients be in the form of complexes. Therefore, the benefits of the present invention can be obtained wherein molecules of at least one active ingredient are complexed with individual molecules ofa water-sensitive complexing agent.

Active ingredients which may be utilized with the present invention includes those previously known and incorporated herein by reference, as well as various pigments, e.g., titanium dioxide, aloe, fragrances, flavorants, menthol, mineral oils, and blends thereof.

It will therefore be appreciated that the various embodiments of the present invention provide shaving aids with novel delivery systems which provide advantages not heretofore realized with previously available shaving aids.

## Claims

1. A shaving aid comprising:
molecules of an active ingredient:
molecules of at least one water sensitive complexing agent which individually complex at least portions of discrete molecules of said active ingredient.

2. A shaving aid according to claim 1 wherein said complexing agent comprises an internal portion and an external portion, and said molecules of said active ingredient complex with said internal portion of said complexing agent.

3. A shaving aid according to claim 1 or claim 2 wherein said complexing agent is a polysaccharide.

4. A shaving aid according to any proceeding claim wherein said complexing agent is a cyclic polysaccharide.

5. A shaving aid according to any proceeding claim wherein said complexing agent is a cyclodextrin.

6. A shaving aid according to any proceeding claim wherein said compexing agent is selected from the group consisting of alph-cyclodextrin, beta-cyclodextrin, gamma cyclodextrin, hydroxypropyl beta cyclodextrin, and mixtures thereof.

7. A shaving aid according to any proceeding claim wherein said active ingredient is oil soluble.

8. A shaving aid according to any proceeding claim wherein said active ingredient comprises Vitamin E.

9. A shaving aid according to any proceeding claim wherein said active ingredient comprises menthol.

10. A shaving aid according to any proceeding claim wherein said active ingredient is selected from the group consisting of lubricating agents, polyethylene oxide, polyacrylamide, depilatory agents, cleaning agents, medicinal agents, cosmetic agents, coagulants, astrigents, vitamins, essential oils, pigments, aloe, fragrances, flavorants, menthol, mineral oils, and blends thereof.

11. A shaving aid according to any proceeding claim wherein said complexing agent is a cyclic polymer.

12. A shaving aid comprising at least one active ingredient and cyclodextrin.

13. A method of forming a shaving aid comprising the steps of:
providing an active ingredient;
providing a water sensitive complexing agent;
contacting said active ingredient with said water sensitive complexing agent to form inclusion complexes; and
incorporating said inclusion complexes into a shaving system.

14. A method of forming a shaving aid according to claim 12 wherein said complexing agent comprises a cyclodextrin.

15. Use of a water sensitive complexing agent in the manufacture of a shaving aid.
